(19) Europäisches Patentamt
European Patent Office
Office européen des brevets

(11) Publication number: 0 200 399
A2

(12) # EUROPEAN PATENT APPLICATION

(21) Application number: 86302663.9

(22) Date of filing: 10.04.86

(51) Int. Cl.[4]: C 09 D 5/44

(30) Priority: 18.04.85 GB 8509956

(43) Date of publication of application:
05.11.86 Bulletin 86/45

(84) Designated Contracting States:
AT BE DE FR GB IT NL SE

(71) Applicant: IMPERIAL CHEMICAL INDUSTRIES PLC
Imperial Chemical House Millbank
London SW1P 3JF(GB)

(71) Applicant: DULUX AUSTRALIA LTD
8, Nicholson Street
Melbourne Victoria(AU)

(72) Inventor: Redman, Richard Paul
10, Earley Hill Road Reading
Berks England(GB)

(72) Inventor: Beresford, Michael Paul
12 Booran Avenue
Glen Waverley Victoria 31500(AU)

(74) Representative: Kerr, Michael Arthur et al,
Imperial Chemical Industries PLC Legal Department :
Patents PO Box 6 Bessemer Road
Welwyn Garden City Herts, AL7 1HD(GB)

(54) **Coating compositions, suitable for electrodepostion, comprise a hydroxyl group-containing addition copolymer and a hydroxyl group-containing polymeric modifier.**

(57) A coating composition which comprises - preferably as an aqueous dispersion of cationically, anionically or non-ionically dispersed particles - (a) a hydroxyl group-containing polymeric modifier, e.g. a polyepoxide resin or a modified polyepoxide resin, (b) a hydroxyl group-containing addition copolymer containing monomer residues of a specified hydroxyl group-containing ester of an ethylenically unsaturated monocarboxylic acid and, preferably, (c) a crosslinking agent for (a) and (b). Cationically stabilised particles are particularly suitable for cathodic electrodeposition from an aqueous medium when the polymeric modifier is an amine-polyepoxide reaction product.

EP 0 200 399 A2

Z/W.33448/EP

0200399

COATING COMPOSITIONS, SUITABLE FOR ELECTRO-DEPOSITION, COMPRISE A HYDROXYL GROUP-CONTAINING ADDITION COPOLYMER AND A HYDROXYL GROUP-CONTAINING POLYMERIC MODIFIER.

This invention relates to crosslinkable coating compositions which comprise a hydroxyl group-containing addition copolymer and a hydroxyl group-containing polymeric modifier.

In our British Patent No. 2 102 436B, we have described aqueous coating compositions which comprise cationically, anionically or non-ionically stabilised particles, each particle comprising:

(a) a polymeric modifier comprising reactive groups,
(b) an addition polymer comprising reactive groups of the same or similar chemical type to the reactive groups in the polymeric modifier, and
(c) a crosslinking agent which comprises groups complementary to the reactive groups present in (a) and (b).

These multi-component particle coating compositions are particularly useful for application to a substrate by electrodeposition. Certain hydroxyl group-containing comonomers are mentioned in BP 2,102,436B as a suitable source of the reactive group in the addition polymer and these include hydroxybutyl acrylate. However, the precise structure of this monomer is not mentioned, its use is not exemplified and the Examples all describe the preparation of the addition polymer in the presence of the polymeric modifier.

We have now found surprisingly that the use of very specific hydroxyl group-containing addition copolymers in multi-component particle coating compositions of this general type provides at least one unforeseen

advantage. When such a coating composition is used to provide on a substrate a primer coating to which is subsequently applied a further coating (i.e. so as to provide a two-layer coating) there is an improvement in the resistance of the two-layer coating to "chipping". The phenomenon of "chipping" is that which may occur on a glancing impact of the coating with an object and is often due to poor adhesion between the two coating layers. Our finding of improved intercoat adhesion is of particular importance in connection with the protection of steel motor car bodies by a coating of two or more layers which is liable to be damaged due to "chipping" by flying stones particularly by gravel in winter driving conditions when salt is also present. Additionally we have found that it is often advantageous to prepare the defined hydroxyl group-containing addition copolymers independently of the polymeric modifier, rather than in situ.

Although the improved compositions are usually aqueous, and then they are particularly suitable for application by electrodeposition when the aqueous composition comprises cationically or anionically stabilised multi-component particles, we have also found surprisingly that there is an improvement in adhesion between two adjacent coating layers when one of the layers is produced from a solution or a dispersion in an organic liquid medium of the same defined hydroxyl group-containing addition copolymer, the polymeric modifier and, optionally, a crosslinking agent.

According to this invention we provide a coating composition which comprises:

(a) 55-95% by weight based on the combined weight of (a) and (b) of a hydroxyl group-containing polymeric modifier as herein defined,

and (b) 5-45% by weight based on the combined weight of (a) and (b) of a hydroxyl group-containing

addition copolymer which comprises :

(i) 30-95% by weight based on the weight of the copolymer of monomer residues of a hydroxyl group-containing carboxylic acid ester of structure:

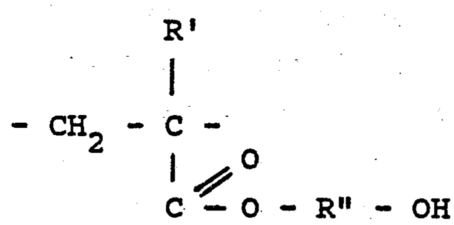

wherein -R' is H or $CH_3$ and -R"- is a $C_{4-18}$ alkylene group or -O-R"- is a (poly)oxyalkylene group, or -R"- has the structure

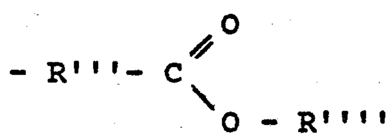

wherein R''' and R'''', the same or different, = $C_{1-8}$ alkyl, -R"- providing a chain of at least 4 atoms which links the hydroxyl group with the acid residue, and

(ii) 5-70% by weight based on the weight of the copolymer of monomer residues which are different from those residues defined in (i).

Preferably the composition also comprises 5-40% by weight, based on the combined weight of (a) and (b), of a crosslinking agent (c).

According to a particular aspect of this invention we provide a coating composition which comprises an aqueous dispersion of cationically, anionically or non-ionically stabilised polymer particles, each particle

comprising:

(a) 55-95% by weight based on the combined weight of (a) and (b) of a hydroxyl group-containing polymeric modifier as herein defined

(b) 5-45% by weight based on the combined weight of (a) and (b) of an addition copolymer which comprises monomer residues as defined above, and optionally

(c) 5-40% by weight, based on the combined weight of (a) and (b), of a crosslinking agent which is reactive with the hydroxyl groups of (a) and (b).

We have found that only when the hydroxyl group of the monomer residue of the hydroxyalkyl ester of an ethylenically unsaturated monocarboxylic acid present in the addition copolymer is separated from the acid residue by a chain of at least 4 atoms, and only when the residue is present in the copolymer in a relatively large amount, is the surprising advantage of improved adhesion between adjacent coating layers achieved.

By a polymeric modifier we mean a polymeric film-forming material which modifies the film-forming properties of the addition copolymer and which itself contributes to the essential properties of the ultimate coating, for example hardness, chemical resistance, corrosion protection and mechanical strength.

Particularly suitable polymeric modifiers are the hydroxyl group-containing polyepoxide resins such as the polyglycidyl ethers of polyphenols, for example those derived from epichlorhydrin and diphenylolpropane, and of molecular weight in the range 340-5000. Specific and useful polyepoxide resins of this type are commercially

available from Shell Chemicals under the Registered Trade Mark "Epikote". The hydroxyl group-containing polyepoxide resins may also be modified, for example in order to improve the properties of the ultimate coating or with a view to achieving cationic, anionic or non-ionic stabilisation of the dispersed polymer particles in an aqueous medium in the aqueous coating compositions of this invention. Thus they may be modified, for example, by reaction of their epoxide groups with amines, with carboxl group-containing compounds or with thiols.

Particularly suitable modified polyepoxide resins include those prepared by reacting epoxide groups in a polyepoxide resin with primary and/or secondary amines. An example of a suitable such reaction product can be prepared by co-reacting a polyepoxide, a polyoxyalkylene polyamine, a monoamine, and optionally a monoepoxide. Modified polyepoxide resins of this type may be dispersed in water, for example by neutralisation of at least some of the secondary amine groups which remain in the resin with an acid such as acetic or lactic acid.

Other suitable modified polyepoxide resins include those in which epoxide groups are reacted with carboxylic acids, for example aliphatic carboxylic acids such as lactic acid and dimethylolpropionic acid or aromatic carboxylic acid such as benzoic acid. The polyepoxide resins so modified may be further modified by reaction with cyclic carboxylic anhydrides as described in British Patent 1,556,201. This further modification will introduce carboxylic groups into the resin. Modified polyepoxide resins of this type may be dispersed in water by neutralisation of at least some of the carboxyl groups introduced into the resin, using a base such as diethanolamine or potassium hydroxide.

The modified polyepoxide resins just described for use as the polymeric modifier in this invention will

contain hydroxyl groups and may also contain other crosslinkable groups, for example amino groups.

Other suitable polymeric modifiers which contain hydroxyl groups and which are useful in this invention include for example, polyester resins, alkyd resins, polyethers, polybutadienes and cellulose esters such as cellulose acetate/butyrate.

The hydroxyl group-containing addition copolymer can be prepared by conventional procedures from a mixture of two or more different monomers, one or more of which will provide in the final copolymer the defined hydroxyl group-containing residues (i) and one or more of which will provide different residues. Preferably the mixture of monomers contains non-reactive monomers which are free from hydroxyl groups, for example butyl acrylate or 2-ethyl hexyl acrylate. Alternatively the addition copolymer used in this invention can be prepared from comonomers at least one of which contains a reactive group, this group being reacted with a coreactive material subsequent to copolymerisation in order to form the defined hydroxyl group-containing residue. One example of such a reactive group is a carboxyl group which may be reacted, after copolymerisation, with a suitable diol.

Suitable momomers to provide the defined monomer residue (i) in the addition copolymer are the hydroxyalkyl esters of ethylenically unsaturated monocarboxylic acids, for example 4-hydroxy-n-butyl acrylate and 4-hydroxy-n-butyl methacrylate. 6-hydroxy n-hexyl acrylate or methacrylate may also be used. Other suitable monomers are the hydroxy(poly)oxyalkylene esters of ethylenically unsaturated monocarboxylic acids, for example where alkylene is ethylene or propylene or mixtures thereof, and these include polypropylene glycol acrylates and methacrylates of structure:

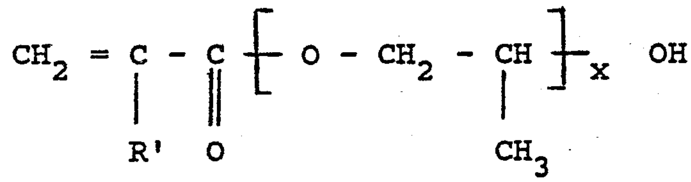

wherein x = 2-50, and R' = H or $CH_3$. Preferably x = 2-10. (Monomers of this type are commercially available from BP Chemicals Limited).

Thus preferred hydroxyl group-containing copolymers are those in which R" is n-butylene or -O-R"- is $-[(O.CH_2 - CH(CH_3))]_x$ where x is an integer from 2 to 50.

Other suitable polymerisable monomers which will provide the defined monomer residue may be prepared by adduction of propylene oxide, butylene oxide and ethylene oxide with polymerisable hydroxylated acrylates or methacrylates. Alternatively hydroxyalkyl(meth)acrylates, e.g. hydroxyethylmethacrylate, may be reacted with a lactone, e.g.caprolactone, to provide a hydroxyalkyl ester of an ester formed from a hydroxy alkanoic acid and an ethylenically unsaturated carboxylic acid. Mixtures of the above-mentioned monomers may be used.

Suitable comonomers which may be used in conjunction with the monomers described above, to provide the different monomer residue (ii) include comonomers which are either substantially non-reactive or which contain a reactive group capable of reaction with another reactive group, for example with a group present in a crosslinking agent. Comonomers which are substantially non-reactive include the $C_{1-18}$ alkyl esters of acrylic and methacrylic acid, for example the methyl ethyl, propyl, butyl, 2-ethyl-hexyl, lauryl and stearyl esters; alkoxy alkyl esters such as ethoxyethyl methacrylate; styrene; acrylonitrile and methacrylonitrile. Comonomers containing reactive groups include carboxyl group-containing monomers such as acrylic, methacrylic, and crotonic acids;

epoxide group-containing monomers such as glycidyl acrylate and methacrylate; amine group-containing monomers, for example dimethyl-amino ethyl methacrylate; and amide group-containing monomers, for example acrylamide, methacrylamide, and n-butoxy-methacrylamide.

The selection of appropriate comonomers in preparing the addition copolymer will be dictated by such factors as their reactivity ratios; the properties which they are required to confer on the copolymer, for example the solubility or dispersibility of the polymer, in specified media for example water or organic liquids; the hardness or softness of the polymer; or a requirement for specific types of group, for example ionisable groups to enable dispersibility in water or reactive groups to enable crosslinking.

Particularly suitable addition copolymers for use with a polyepoxide-amine reaction product as the polymeric modifier, or with modified polyepoxides prepared according to British Patent 1,556,201 mentioned above, include 2-component copolymers of 4-hydroxy-n-butyl(meth)acrylate or polypropylene glycol acrylate or methacrylate with butyl acrylate, butyl methacrylate, methyl methacrylate, and 2-ethylhexyl acrylate. Ter- or even higher-component copolymers may be used.

There must be present in the addition copolymer at least 30% by weight of the defined monomer residue, based on the weight of the copolymer, but preferably there is present 35-90% by weight of such a residue, more preferably 50-80%.

In general the crosslinking agent, which is preferably present in the coating compositions of this invention, will be one which is reactive with hydroxyl groups, for example capped or blocked polyisocyanates; β-hydroxyesters of the type described in European Patent 0 040 867; or phenol-, melamine-, urea-, benzoguanamine-or glycoluril-formaldehyde resins. However, if types of crosslinkable group other than hydroxyl are also present in either (a) or (b), other suitable crosslinking agents may be used.

Suitable polyisocyanates include mixtures of 2,4- and 2,6-toluene diisocyanate; and hexamethylene diisocyanate. A proportion of the isocyanate groups, for example half, may be reacted with an alcohol, for example an alkoxy alcohol, and the remaining isocyanate groups reacted with, for example, trimethylolpropane to obtain a crosslinking agent which is reactive with hydroxyl groups only at elevated temperatures.

The coating compositions of this invention may comprise a dispersion or a solution of the hydroxyl group-containing polymeric modifier (a), the hydroxyl group-containing addition copolymer (b) and optionally the crosslinking agent (c) in an organic liquid medium. Such compositions are usually prepared by combining the three separate components in the required medium and may be applied to a substrate, for example, by spraying.

Preferably, and bearing in mind the general preference to use water-based coating compositions for environmental reasons, the coating compositions of this invention comprise a dispersion in an aqueous medium of particles which each comprise the hydroxyl group-containing polymeric modifier (a), the hydroxyl group-containing addition copolymer (b), and optionally the particles also comprise the crosslinking agent (c). By an aqueous medium we mean a liquid medium which comprises at least 25% and preferably at leaast 50% by weight of water. The disperse particles may be cationically, anionically or non-ionically stabilised and the stabilisation may be due to stabilising groups present in the polymeric modifier (a), in the hydroxyl group-containing addition copolymer (b), or to stabilising groups

present in a surfactant (distinct from the polymer modifier and the addition copolymer) which is associated with the particles.

When the disperse particles are cationically stabilised in an aqueous medium stabilisation may be due to the presence of basic groups, for example, primary secondary or tertiary amino groups, in the polymeric modifier or in the addition copolymer at least 30% of which, and up to 100% of which, are neutralised by an acid such as acetic acid or lactic acid. As mentioned above, suitable amino groups may be introduced into a polyepoxide-based polymeric modifier by reaction of its epoxide groups with a monoamine or with a polyamine. Similarly suitable basic groups may be introduced into the addition copolymer by the selection of a suitable comonomer. Suitable distinct cationic surfactants which may be used to stabilise the polymer particles include fatty amine salts, for example a lower alkyl salt of a fatty mono- or di-amine. Addition copolymer surfactants may also be used.

When the disperse particles are anionically stabilised in an aqueous medium, stabilisation may be due to acidic groups, for example carboxyl groups, in the polymeric modifier or in the addition copolymer at least 30% of which, and up to 100% of which, are neutralised by a base such as an amine or an alkali metal hydroxide. As mentioned above, carboxyl groups may be introduced into a polyepoxide polymer modified by the process described in British Patent 1,556,201. Similarly carboxyl groups may be introduced into the addition copolymer by the selection of a suitable comonomer, for example (meth)acrylic acid. Suitable distinct anionic surfactants which may be used to stabilise the polymer particles include for example the amine or alkaline metal salts of fatty acids such as oleic

acid or stearic acid, for example the triethanolamine salt; or the alkyl aryl sulphonates.

When the disperse particles are non-ionically stabilised in an aqueous medium, stabilisation may be due to water-soluble non-ionic groups, for example a group comprising a polyethylene oxide condensate, which may be present in the polymer modifier or in the addition copolymer. When the polymer modifier is, for example, a polyepoxide resin, suitable non-ionic groups may be introduced by reaction of epoxide groups with a poly-(ethylene glycol). Similarly, water-soluble non-ionic stabilising groups may be introduced into the addition copolymer by the selection of a suitable comonomer for example a meth(acrylate) ester of a poly(ethylene glycol).

The aqueous coating compositions according to the invention may be prepared by a variety of processes. In a preferred process the polymer modifier and the addition copolymer are prepared separately, for example in the presence of miscible organic solvents, and are then mixed, together with the crosslinking agent when this is to be present. The mixture in organic solvent is then emulsified in water and the organic solvent is volatilised to yield an aqueous dispersion of polymer particles according to the invention.

Coating compositions according to the invention may comprise ingredients other than the polymer modifier, the defined addition copolymer and the crosslinking agent. These additional ingredients may be present within or without the disperse polymer particles. For example, there may be present other polymeric ingredients, such as addition polymers different from the specific addition copolymers defined above. There may also be present ingredients commonly present in coating compositions, for example pigment, filler and coalescing solvent.

Coating compositions according to the invention

may be applied by a wide variety of processes, for example by dipping, spraying and electrodeposition to a wide variety of substrates. Suitable substrates are metals and plastics.

However the coating compositions are particularly suitable for the application of primer coatings to a metal substrate by electrodeposition and according to a further aspect of this invention we provide a process of coating a conductive article by electrodeposition which consists in immersing the article as one electrode in a coating composition comprising an aqueous dispersion of cationically or anionically stabilised particles as defined herein and passing an electric current between the article and a counter-electrode in electrical contact with the composition.

The conditions of electrodeposition are according to conventional practice.

The invention is illustrated by the following Examples in which parts and percentages are by weight unless otherwise stated :

EXAMPLES 1-8

These Examples illustrate the preparation of eight different addition copolymers containing hydroxyl groups (see Table I), and their use, respectively, as one component of multi-component particle coating compositions according to the invention.

(a) Preparation of hydroxyl group-containing copolymers

Each of the copolymers was prepared in a similar manner as is now illustrated for Example 3.

11 parts of azobisisobutyronitrile were dissolved in a mixture of 255 parts of 4-hydroxy-n-butyl acrylate and 85 parts of butyl acrylate containing 1.5 parts of octyl mercaptan. The mixture was passed over 3 hours into a flask containing 150 parts of methyl isobutyl ketone at the reflux temperature. Reflux was maintained for a further 1 hour, 0.7 parts of azobisisobutyronitrile in 3 parts of methyl isobutyl ketone were added and heating at the reflux temperature continued for a further 2 hours. The resulting solution (70% solids) of a 75/25 copolymer of 4-hydroxy-n-butyl acrylate/butyl acrylate had a Gardner Holdt viscosity of v. The weight average molecular weight of the polymer (determined by gel phase chromatography) was about 12,000 and the free monomer content was less than 0.5% as determined by gas-liquid chromatography.

(b) Preparation of coating compositions and application to a substrate by electrodeposition

Each of the addition copolymers prepared as described in (a) was mixed with a polyepoxide-amine reaction product (the polymeric modifier) prepared as follows:

2000 parts of a linear polyoxypropylene diamine of molecular weight 400 which had terminal primary amino-containing groups of structure

$H_2N - CH(CH_3)-CH_2-O-$

(commercially available from the Jefferson Chemical Company as "Jeffamine" D400) were mixed with 120 parts of the glycidyl ester of a $C_8$ tertiary alkyl carboxylic acid (commercially available from Shell Chemicals as "Cardura" E) and heated to 100°C. After the exotherm the temperature was maintained at 115°C for about 90 minutes when the epoxy value had fallen to approximately zero. The product had an amine value of 2.81 millimoles/g.

1714 parts of a diepoxide (commercially available from Shell Chemicals as "Epikote" 1004) ["Epikote" is a Registered Trade Mark] and of epoxide equivalent 930 were dissolved in 1051 parts of methylisobutyl ketone by heating to 90°C with vigorous stirring. The solution had an epoxide value of 0.67 millimoles/g. 447 parts of the epoxide-amine reaction product prepared above and 85.8 parts of methyl ethanolamine were added to the solution of the diepoxide and the mixture was maintained at the reflux temperature for 2 hours. The epoxide value had then fallen to 0.01 millimoles/g; the amine value was 0.74 millimoles/g; and the solids content of the product was 68% by weight.

The coating compositions corresponding to each of the eight addition polymers were prepared by mixing 900 parts of the above epoxide-amine reaction product; 180 parts (of 70% solids content) of the addition copolymer prepared in (a); 240 parts of a cross-linking

agent in the form of a 70% solids solution in methylisobutyl ketone obtained by reacting half of the isocyanate groups in an 80/20 mixture of 2,4- and 2,6-toluene diisocyanate with 2-butoxyethanol, and then reacting 3 parts of the product with 1 part of trimethylolpropane; 30 parts of phenoxypropanol as coalescing solvent; and 42 parts of an 80% by weight aqueous solution of lactic acid. 2400 parts of water were added to this mixture slowly and with vigorous agitation using a Silverson emulsifier.

The resulting emulsion was stirred and heated under inert gas to remove methyl isobutyl ketone, any other volatile organic solvent present, and some water. The water removed was subsequently replaced, and after filtration the resulting dispersion was diluted to 20% solids. A conventional pigment millbase containing a tin catalyst was then added to produce a coating composition.

A phosphated steel panel as cathode was coated by electrodeposition at 350 volts for 2 minutes using the above coating composition at a bath temperature of 27°C. The composition had a pH of 5.6 and a conductivity of 1650 μs $ccm^{-1}$. The panel was removed, rinsed with demineralised water and stoved at 180°C for 20 minutes. Smooth corrosion-resistant coating films were produced.

(c) <u>Application of a topcoat to electrodeposited coatings and mechanical testing of the final coating system</u>

To each of the panels coated by electrodeposition as described in (b) above was applied a topcoat. Two panels were coated by electrodeposition in each case corresponding to each of the eight hydroxyl group-containing copolymers shown in Table I. Each of these two panels was topcoated with a commercially available paint, one being based on an alkyd/melamine formaldehyde resin and the other on an acrylic/melamine

formaldehyde resin. The topcoated panels were stoved at 135°C for 20 minutes.

The panels, which were now coated with a coating system comprising an electrodeposited primer coating and a topcoat comprising either an alkyd/melamine-formaldehyde resin or an acrylic/melamine-formaldehyde resin, were subjected to mechanical testing to determine chip resistance and intercoat adhesion (see Table I and test methods described below in which the figures quoted are an average of the results obtained for both types of topcoat).

The performance of these panels was surprisingly better than the performance of the panels prepared in Examples 9-16.

## EXAMPLES 9-15

These Examples relate to the preparation of certain copolymers which contain hydroxyl groups and certain copolymers which are free from hydroxyl groups (see Table), and, to their separate use as one film-forming component of multi-component coating compositions which comprise as the other film-forming components an epoxide-amine reaction product and a crosslinking agent. These compositions are not in accordance with the invention.

The copolymers were prepared in the manner described above in (a) of Examples 1-8. Likewise the epoxide-amine reaction product, the coating composition and the final coating systems were prepared as described in (b) and (c) of Examples 1-8 above. Both the Chip rating and the Intercoat adhesion rating of these coating systems were unsatisfactory.

## EXAMPLE 16

When no addition copolymer was present in the coating composition applied by electrodeposition the Chip resistance rating was 2.0 - 2.5 and the Intercoat adhesion rating was greater than 80%, i.e. unsatisfactory.

KEY TO TABLE

PPM 6E +++ = a polypropylene glycol methacrylate of the structure 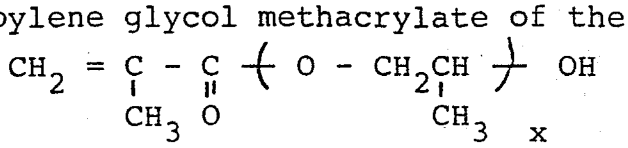

where x on average is 6

(commercially available from B.P.Chemicals as "Bisomer PPM6E")

PPM 5S ++ = similar to PPM 6E but with x = 5

BA = butyl acrylate

HBA + = 4-hydroxy-n-butyl acrylate

EHA = 2-ethylhexyl acrylate

BMA = butyl methacrylate

MMA = methyl methacrylate

HEA = hydroxyethyl acrylate

HEMA = hydroxyethyl methacrylate

HIPMA = hydroxyisopropyl methacrylate

Tg (°C) : the values are approximate and are calculated from the published Tg values of the homopolymers using the equation $^{1}/Tg = {}^{W_1}/Tg + {}^{W_2}/Tg$ where $W_1$ and $W_2$ are the weight fractions of the polymers having $Tg_1$ and $Tg_2$ glass transition temperatures.

+ 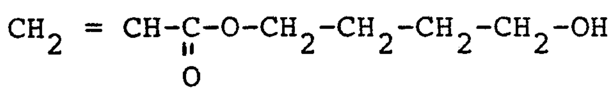 chain of 4 atoms separates hydroxyl group from acid residue

++ 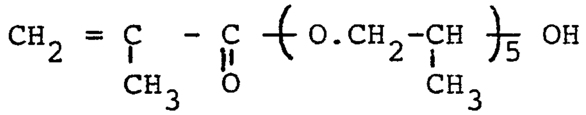 chain of 14 atoms "

+++ 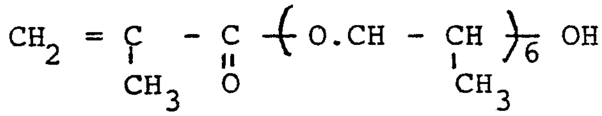 " 17 " "

Determination of "chip rating" and Intercoat adhesion Rating

Five lots, each of 500g, of split steel shot are directed onto the painted panels using compressed air at 3 bar. The panels are mounted at 45°C to the direction of movement of the shot and the shot covers a panel area of about 8cm x 8cm. Paint chipping is induced, some chips reaching to the steel substrate and others only reaching the primer layer,

TABLE I

| Example No. | Hydroxyl group-containing copolymer | | | | | Final coating system | |
|---|---|---|---|---|---|---|---|
| | Comonomers * | Comonomer ratio | Number of atoms separating hydroxyl group from ester residue | Hydroxyl content (mole %) | Glass trans-ition temp.(°C) | Chip Rating ** | Intercoat adhesion. Rating (%) *** |
| 1. | PPM 6E/BA | 75/25 | 17 | 2.9 | v.low | <1 | < 20 (Examples 1–2) |
| 2. | PPM 5S/BA | 75/25 | 14 | 3.4 | v.low | <1 | |
| 3. | HBA/BA | 75/25 | 4 | 8.8 | -37 | 1 - 1.5 | 20-40 (Examples 3–4) |
| 4. | HBA/EHA | 75/25 | 4 | 8.8 | -47 | 1 - 1.5 | |
| 5. | HBA/EHA | 50/50 | 4 | 5.9 | -61 | 1 - 1.5 | 40-60 (Examples 5–8) |
| 6. | HBA/BA | 50/50 | 4 | 5.9 | -44 | 1 - 1.5 | |
| 7. | HBA/BMA | 50/50 | 4 | 5.9 | -7 | 1 - 1.5 | |
| 8. | HBA/MMA | 50/50 | 4 | 5.9 | +21 | 1.5 | |
| 9. | BMA/BA | 75/25 | - | - | -3 | 1.5 | 60-80 (Examples 9–13) |
| 10. | BMA/EHA | 30/70 | - | - | -61 | 1.5-2.0 | |
| 11. | HEA/EHA | 60/40 | 2 | 8.8 | -49 | 2.0-3.0 | |
| 12. | HEMA/BA | 75/25 | 2 | 9.8 | +17 | 1.5 | |
| 13. | HEA/BA | 75/25 | 2 | 11.0 | -26 | 1.5 | |
| 14. | HEA/EHA | 40/60 | 2 | 5.8 | -61 | > 4.0 | > 80 (Examples 14–15) |
| 15. | HIPMA/BA | 75/25 | 2 | 8.8 | +28 | 2.0-2.5 | |

* see Key to comonomers used

** Visual Chip rating scale : 1.0, 1.5, 2.0, 2.5, 3.0, 4.0
→ increasing severity of chips which penetrate the electrodeposited primer.

*** % of chips in which the primer layer remains intact, i.e. loss of adhesion between primer layer and topcoat.

i.e. in the latter case there has been intercoat adhesion failure. To highlight the failures down to metal the steel panels are dipped into 5% acidified copper sulphate solution for 10 minutes. After rinsing, the chips which reach the metal are apparent as bright copper spots.

The total chip failure is then visually assessed as a Chip Rating using a scale of 1-4 (see above). The failures in adhesion which occur between the primer-topcoat interface are assessed visually. They are expressed as a percentage of the total chip failure and in the Table are quoted in 20% bands.

EXAMPLE 17

This Example illustrates the invention with respect to four types of topcoat in both 2-coating layer and 3-coating layer systems.

The procedure of Examples 3 (a) and (b) was followed and the electrodeposited, stoved coating on individual members of a set of panels was:-

a) directly overcoated with :-

1. a commercially available alkyd/melamine-based solid colour topcoat.
2. a commercially available acrylic/melamine-based solid colour topcoat.
3. a commercially available solvent-borne aluminium-containing basecoat/clear system.
4. a commercially available water-borne aluminium-containing basecoat and solvent-borne clear-coat.

b) first coated with a commercially available polyester-based solvent-borne surfacer and stoved; and then further coated with each of the topcoats 1-4 listed above.

The resulting panels were subjected to the Chip rating and Intercoat adhesion test described in connection with Table 1 and the results are given in Table 2.

TABLE 2

| Example 17 | Chip Rating | Intercoat Adhesion Rating (%) |
|---|---|---|
| a) 1 | 1.0 | < 20 |
| 2 | 1.0 | |
| 3 | 1.0-1.5 | 20-40 |
| 4 | 1.0-1.5 | 40-60 |
| b) 1 | 1.0 | < 20 |
| 2 | 1.0 | |
| 3 | 1.0 | 20-40 |
| 4 | 1.0 | < 20 |

EXAMPLE 18

This Example illustrates (a) the preparation of a modified polyepoxide resin, the modified resin containing carboxylic groups which enable dispersion of the resin in an aqueous medium in the presence of a base and (b) the use of this resin in coating metal panels by spray application when combined with a hydroxyl group-containing copolymer and a crosslinking agent to produce an anionically-stabilised dispersion of particles according to the invention.

a) "Epikote" 1001 (1800 parts) was dissolved in methyl-isobutyl ketone (800 parts) at 90°C in a flask fitted with a Dean & Stark separator. Adipic acid (116 parts) dimethylol propionic acid (212 parts) and dimethylbenzylamine (5 parts) was added and the mixture refluxed at 145°C. Any water in the mixture was removed by the Dean & Stark separator. After 3 hours the acid value had fallen to 0.01 mm/g.

The product was cooled to 100°C and succinic anhydride (160 parts) added. The mixture was maintained at 120°C for a further 2 hours. Methyl isobutyl ketone (375 parts) was added to give a solution of 66% solids content.

b) 140 parts of the above resin solution were added to 40 parts of the 70% solids solution of acrylic copolymer described in Example 3, 20 parts of a urea formaldehyde resin (commercially available from American Cyanamid as UFR 80) and 4 parts of dimethylethanolamine. The mixture was emulsified with 250 parts of water.

Metal panels were sprayed with the above emulsion and stoved at 160°C for 30 minutes. Smooth corrosion-resistant films were obtained.

Separate panels thus coated were topcoated with a commercially available alkyd/melamine-based paint and a commercially available acrylic/melamine-based paint respectively.

When subjected to the stone chip test a good

Chip rating and Intercoat adhesion rating were recorded.

EXAMPLE 19

This Example illustrates the preparation and use of a solution in organic solvent of a coating composition according to the invention.

A modified polyepoxide resin containing carboxyl groups was prepared as described in Example 18(a). 140 parts of this resin (as a solution of 66% solids) were added to 40 parts of the acrylic copolymer (as a solution of 70% solids) used in Example 3, and 20 parts of a ureaformaldehyde resin (UFR 80). The viscosity of the mixture was adjusted by the addition of butyl cellosolve to give a viscosity of 25 seconds in a B4 Ford cup.

Panels were sprayed and stoved and subsequently topcoated as in Example 17. Similar good Chip resistance rating and Intercoat adhesion rating were observed.

Z/PV 33448

CLAIMS :

1. A coating composition which comprises :

(a) 55-95% by weight based on the combined weight of (a) and (b) of a hydroxyl group-containing polymeric modifier as herein defined,

and

(b) 5-45% by weight based on the combined weight of (a) and (b) of a hydroxyl group-containing addition copolymer which comprises

(i) 30-95% by weight based on the weight of the copolymer of monomer residues of a hydroxyl group-containing carboxylic acid ester of structure :

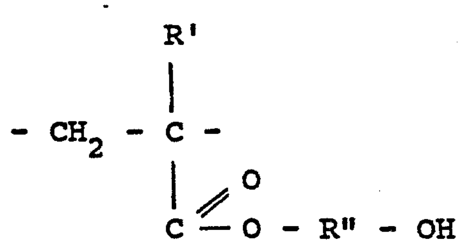

wherein R' is H or $CH_3$ and R" is an alkylene group or -O-R"- is a (poly)oxyalkylene group, or -R"- has the structure

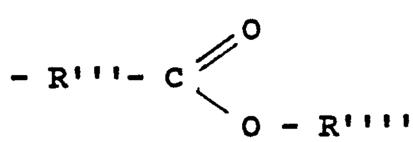

wherein R''' and R'''', the same or different, = $C_{1-8}$ alkyl, -R"- providing a chain of at least 4 atoms which links the hydroxyl group with the acid residue, and

(ii) 5-70% by weight based on the weight of the copolymer of monomer residues which are different from those defined in (i).

2. A coating composition according to claim 1 which also comprises :

   (c) 5-40% by weight, based on the combined weight of (a) and (b), of a crosslinking agent which is reactive with the hydroxyl groups of (a) and (b).

3. A coating composition according to claim 1 or claim 2 wherein -R"- is an n-butylene group or -O-R"- is

$$-\!\!\left[\,O.CH_2\text{-}\underset{CH_3}{CH}\,\right]\!\!-_x$$

and wherein x is an integer from 2 to 50.

4. A coating composition according to any one of claims 1 to 3, wherein the monomer residues (ii) are free from hydroxyl groups.

5. A coating composition according to any one of claims 1 to 4, wherein the polymeric modifier is a polyepoxide resin or a modified polyepoxide resin.

6. A coating composition according to any one of claims 1 to 5, wherein the modified polyepoxide resin is a polyepoxide-amine reaction product.

7. A coating composition according to any one of claims 1 to 6, which comprises an aqueous dispersion of cationically, anionically or non-ionically stabilised polymer particles, each particle comprising:

   (a) 55-95% by weight based on the combined weight of (a) and (b), of the hydroxyl group-containing polymeric modifier.

   (b) 5-45% by weight based on the combined weight of (a) and (b) of the hydroxyl group-containing addition copolymer, and optionally

   (c) 5-40% by weight based on the combined weight of (a) and (b) of a crosslinking agent which is reactive with the hydroxyl groups of (a) and (b).

8. A coating composition according to claim 7 wherein the disperse particles are cationically stabilised by the neutralisation with an acid of at least 30% of the amino groups in the polymeric modifier.

9. A coating composition according to claim 8 wherein the polymeric modifier is a polyepoxide-amine reaction product.

10. A process of coating a conductive article by electrodeposition which consists in immersing the article as one electrode in a coating composition comprising an aqueous dispersion of cationically or anionically stabilised particles according to claim 7 and passing an electric current between the article and a counter-electrode in electrical contact with the composition.